# EUROPEAN PATENT APPLICATION

(11) **EP 4 332 225 A1**
(43) Date of publication of application: **06.03.2024**
(21) Application number: 22795029.2
(22) Date of filing: 29.04.2022
(51) Int. Cl.: C12N 15/117, A61K 39/39, A61P 37/04, A61P 35/00, A61P 31/00, A61P 37/08

(54) **APPLICATION OF ARTIFICIALLY SYNTHESIZED CPG SINGLE-STRANDED DEOXYOLIGONUCLEOTIDE IN VACCINES**

(30) Priority: 30.04.2021 CN 202110479157
(71) Applicant: Parr Biotechnology (Hebei) Co., Ltd., Shijiazhuang, Hebei (CN)
(72) Inventor: WANG, Ligong, Wuxi, Jiangsu 214142 (CN); CHEN, Yan, Wuxi, Jiangsu 214142 (CN); ZENG, Ting, Wuxi, Jiangsu 214142 (CN)
(74) Representative: Altmann Stößel Dick Patentanwälte PartG mbB
(86) International application number: PCT/CN2022/090392
(87) International publication number: WO 2022/228560

(57) **Abstract**

The disclosure provides a composition comprising CpG ODN and one or more other adjuvants that work together with the immunomodulatory CpG ODN, such as an aluminum adjuvant, wherein the CpG ODN comprises or consists of a nucleotide sequence selected from SEQ ID NOs: 1-4, wherein at least one nucleotide in the nucleotide sequence is a chemically modified nucleotide having a structure of formula (I): 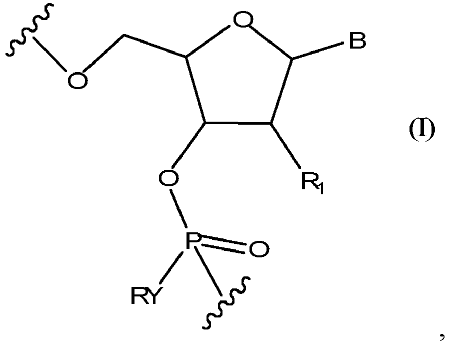 wherein, Y is S or O, R is H or a positively charged counterion, B is independently an unmodified or modified nucleobase, and Ri is H, F, Cl, OH, OMe, Me, O-ethyloxymethyl.

Use of the composition, a pharmaceutical composition comprising the composition, a preparation method thereof, and use of the pharmaceutical composition are also disclosed.

## Description

### FIELD OF THE INVENTION

The present disclosure relates to application of a series of artificially synthesized CpG single-stranded deoxyoligonucleotides in vaccines. Furthermore, the present disclosure relates to application of the artificially synthesized CpG single-stranded deoxyoligonucleotide as a vaccine adjuvant. Furthermore, the present disclosure relates to application of the artificially synthesized CpG single-stranded deoxyoligonucleotide in genetically engineered vaccines, subunit vaccines and inactivated vaccines.

### BACKGROUND OF THE INVENTION

A vaccine is an antigen that is injected into the bloodstream to stimulate the immune system to synthesize antibodies. The application of vaccines plays a very important role in controlling the epidemic of human and animal diseases. According to the principle of vaccine preparation, vaccines can be divided into inactivated vaccines, attenuated vaccines, subunit vaccines and genetically engineered vaccines. Genetically engineered vaccines mainly include genetically engineered subunit vaccines, genetically engineered vector vaccines, nucleic acid vaccines, gene-deleted live vaccines, protein engineered vaccines, etc. Vaccines in a broad sense also include genetic recombinant vaccines, synthetic peptide vaccines, anti-idiotypic antibody vaccines, and microencapsulated controlled sustained-release vaccines.

Recombinant vaccines refer to vaccines produced through genetic recombination mechanisms. In order to solve the problems of traditional vaccines, reduce immunogenicity, improve safety, and reduce treatment period, a new SIT method, i.e., immune recombinant vaccine is used. There are three types: one is DNA recombinant vaccine. The first vaccine launched in this way is hepatitis B vaccine, wherein the hepatitis B surface antigen HBsAg is clonely amplified and the vaccine is produced from yeast by using recombinant DNA technology. The second type of vaccine is a vaccine produced by eliminating and modifying known pathogenic genes on pathogenic microorganisms. The first-generation recombinant vaccine against rotavirus developed in this way has been undergoing clinical trials in the United States and Finland, and research results suggest that the vaccine has strong protection against diarrhea in children caused by rotavirus. The third type of vaccine is a vaccined prepared by inserting a gene of a pathogenic microorganism into a non-pathogenic microorganism such as a virus, and then the modified virus acts as a carrier or vector to express the foreign gene, thereby inducing an immune response. This technology is being used in the development of HIV vaccines. It is also used in the development of vaccines against SARS-CoV-2.

New vaccines such as synthetic peptide vaccines, genetically engineered subunit vaccines, anti-idiotypic antibody vaccines and nucleic acid vaccines have advantages such as good antigenicity and low toxicity, but they have weak immunogenicity and need to be used with efficient adjuvants. These adjuvants include antigen micronization adjuvants (insoluble aluminum salt colloids, immunostimulatory complexes, liposomes), slow-release antigen adjuvants (oil emulsion adjuvants, microencapsule of antigens), microbial adjuvants (bacterial toxins, Corynebacterium parvum vaccine, mycobacteria and their components, peptidoglycan), molecular adjuvants (cytokines, C3d molecules, costimulatory molecules, superantigens, heat shock proteins, CpG sequences), and other adjuvants (vitamin E and selenium, antibiotics, thymomimetic drugs, propolis, traditional Chinese medicine polysaccharides). These adjuvants mainly work through immune regulation, participation in antigen presentation, induction of CD8⁺ T cell responses, and antigen storage.

Synthetic oligodeoxynucleotides containing cytosine-guanine dinucleotide motifs (CpG oligodeoxynucleotide, CpG ODN) can stimulate the natural immune system of higher animals, activate immune cells such as B cells, macrophages (Mϕ), dendritic cells (DC) and NK cells, and induce the secretion of cytokines such as IL-2, IL-6, IL-12, and IFN-γ. CpG ODN is used as a vaccine adjuvant to induce a strong Th1-type immune response required in therapeutic vaccines for various diseases, and has been well verified in humans and mice. Studies in animals have demonstrated that immune defenses established by CpG ODN alone or as vaccine adjuvants protect against a variety of viral, bacterial and parasitic diseases. CpG ODN can be used alone as a vaccine adjuvant, and studies have reported that CpG DNA can be used as an effective enhancer of specific immunization in mice immunized with recombinant hepatitis B surface antigen (Heather L. Davis, etc., J Immunol 1998; 160: 870-876;). CpG-containing single-stranded deoxynucleotides are used as an adjuvant for rabies vaccines, which can significantly enhance the immunization effect of rabies vaccines and reduce the number of injections of rabies vaccines (CN1781929A). The results of the Phase III clinical trial of CpG ODN 1018 ISS enhanced hepatitis B virus vaccine (HEPLISAV) developed by Dynavax showed that subjects in the HEPLISAV group produced protective antibodies at similar levels to those in the Engerix-B group of GlaxoSmithKline's hepatitis B vaccine (Zhao Yujiao, et al., Chinese Journal of Biological Products, Volume 29, Issue 7, July 2016). In addition, CpG ODN can also be used in combination with other adjuvants as a vaccine adjuvant. Clinical trial studies have reported that the immune-stimulating TLR9 agonist oligodeoxynucleotide CPG 7909, as an adjuvant for Engerix-B, enhanced vaccine immunogenicity (C.L. COOPER, etc., Journal of Clinical Immunology, Vol. 24, No. 6, November 2004). The antibody response was significantly enhanced when the adjuvant CPG 7909 was added to the blood-stage malaria vaccine candidate AMA1-C1/Alhydrogel (Vaccine. 2009 June 24; 27(31): 4104-4109). These studies found that CpG-ODNs alone or in combination with other adjuvants (such as aluminum hydroxide, ISA-51, MF59, ISCOMATRIX, IL-2, etc.) could significantly enhance antigen-specific humoral and cellular immune responses. CpG-ODNs can not only enhance the "quantity" of antigen-specific immune responses, but also change the "quality" of antigen-specific immune responses. As far as humoral immunity is concerned, CpG-ODNs can not only accelerate antibody production, increase antibody titers, improve antibody avidity and enhance immune persistence, but also change antibody subtypes (Chinese Journal of New Drugs 2014, 23(1)).

COVID-19 is an emerging infectious disease that is spreading globally. According to the WHO official website, as of December 15, 2020, there have been 71,581,532 confirmed cases of COVID-19 and 1,618,374 deaths. COVID-19 is caused by a new coronavirus (severe acute respiratory syndrome coronavirus 2 (SARS-CoV-2)). The spike (S) protein of SARS-CoV-2, composed of two subunits, S1 and S2, plays a key role in receptor recognition and cell membrane fusion. The S1 subunit contains a receptor-binding domain that recognizes and binds to the host receptor angiotensin-converting enzyme 2, while the S2 subunit mediates viral cell membrane fusion by forming a six-helix bundle through two heptad repeat domains. The fundamental role of the S protein in viral infection suggests that it is a potential target for vaccine development, antibody blocking therapies, and small molecule inhibitors. Potential drugs targeting the S protein include SARS-CoV-2 S protein-based antibodies, fusion inhibitors, and protease inhibitors targeting the SARS-CoV-2 S cleavage site [Letko M, Marzi A, Munster V. Functional assessment of cell entry and receptor usage for SARSCoV-2 and other lineage B betacoronaviruses. Nat Microbiol. 2020;5:562-9.].

Adjuvant technology has been further confirmed and applied in the development of vaccines against SARSCoV-2. By enhancing the immune response and improving immune intensity, adjuvants objectively reduce the amount of antigen required per unit of vaccine, thereby increasing vaccine production capacity and expanding the number of immunized people, and thus will play an immeasurable role in the battle between humans and viruses. So far, at least 10 drug developers have expressed plans to develop adjuvant vaccines against COVID-19, including companies such as British GlaxoSmithKline (GSK), Australian Seqirus and American Dynavax. The above-mentioned companies have committed to develop approved adjuvants (AS03, MF59 and CpG 1018 respectively) and provide them to other people developing vaccines against COVID-19. There are currently multiple adjuvanted vaccines in clinical trials, such as full-length recombinant SARS CoV-2 glycoprotein nanoparticle vaccines containing Matrix-M adjuvant developed by Novavax, Recombinant protein (RBD dimer) vaccines expressed in CHO cells containing adjuvant jointly developed by Anhui Zhifei Longcom Biopharmaceutical Co., Ltd./Institute of Microbiology, Chinese Academy of Sciences, plant-derived VLP vaccines containing AS03 adjuvant developed by Medicago, adjuvant-containing DNA plasmid vaccines jointly developed by Osaka University/AnGes/Takara Bio, adjuvant-containing protein subunit (RBD) vaccines developed by Biological E Ltd, Adenovirus Type 5 (Ad5) adjuvanted oral vaccine platforms developed by Vaxart, recombinant spike protein vaccines containing Advax adjuvant jointly developed by Vaxine Pty Ltd/Medytox, molecular clamp-stabilized spike protein vaccines containing MF59 adjuvant jointly developed by the University of Queensland/CSL/Seqirus, S-2P protein + CpG 1018 vaccines jointly developed by Medigen Vaccine Biologics Corporation/NIAID/Dynavax, and RBD + adjuvant vaccines developed by Cuba's Instituto Finlay de Vacuns. There are also patent documents disclosing vaccines with adjuvants. For example, CN111956797A discloses the application of a new vaccine adjuvant SF (chemically modified cyclic dinucleotide) in vaccines against COVID-19; CN111991556A discloses a SARS-CoV-2 RBD conjugated nanoparticle vaccine added with Sigma Adjuvant System and/or AddaVax; CN111603556A discloses a subunit nanovaccine against COVID-19 containing CpG oligodeoxynucleotides.

Phase III data from the Pfizer-BioNTech COVID-19 vaccine showed a higher incidence of lymphadenopathy in the vaccine group compared with placebo, including 64 subjects in the vaccine group and 6 subjects in the placebo group, which might be related to the vaccine since local lymph nodes were involved in the vaccine response. In addition, there was an imbalance in Bell's palsy among vaccine recipients, including 4 subjects in the vaccine group and 0 subject in the placebo group, the cause of which was unknown. It can be seen that the safety issues of vaccines need further research and resolution.

### SUMMARY OF THE INVENTION

The present disclosure provides CpG ODNs with an immunoregulatory effect. The structures of these CpG ODNs are novel, and they have immunostimulatory effects on both mice and humans and have synergistic effects when used in combination with aluminum adjuvants, and thus are of great clinical value. Specifically, the present disclosure solves the problems in the art through the following technical solutions:
1. A composition comprising CpG ODN and one or more other adjuvants that work together with the immunomodulatory CpG ODN, such as an aluminum adjuvant, such as an aluminum hydroxide and an insoluble aluminum salt colloid, an oil-water emulsion, a microorganism and a metabolite thereof, a nucleic acid and an analog thereof, a cytokine, an immunostimulatory complex, a propolis, and a liposome, preferably an aluminum adjuvant, wherein the CpG ODN comprises or consists of a nucleotide sequence selected from SEQ ID NOs: 1-4, wherein at least one nucleotide in the nucleotide sequence is a chemically modified nucleotide having a structure of formula (I): 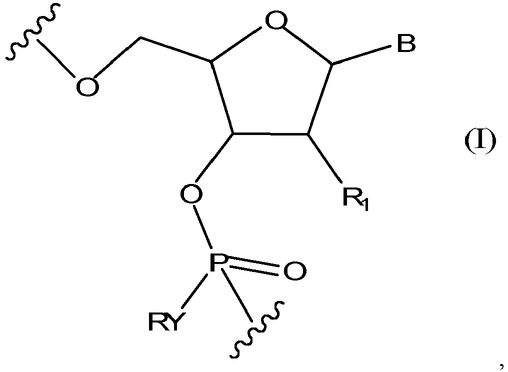 wherein, Y is S or O, R is H or a positively charged counterion, B is independently an unmodified or modified nucleobase, and R₁ is H, F, Cl, OH, OMe, Me, O-ethyloxymethyl.
2. The composition of item 1, wherein Y is S.
3. The composition of item 1 or 2, wherein all nucleotides in the nucleotide sequence of the CpG ODN are chemically modified nucleotides having the structure of formula (I).
4. The composition of item 3, wherein the sequence of the CpG ODN is selected from SEQ ID NOs: 1-4, preferably, completely phosphorothioated SEQ ID NOs: 1-4.
5. The composition of any one of items 1-4, wherein the amount of CpG ODN is 10-500 µg/mL, and the amount of aluminum in the aluminum adjuvant is 300-600 µg/mL.
6. Use of the composition of any one of items 1-5 in the preparation of a vaccine.
7. The use of item 6, wherein the vaccine is a hepatitis B vaccine, a rabies vaccine, a COVID-19 vaccine, such as a COVID-19 vaccine against SARS-CoV-2 Alpha strain, Beta strain, Gamma strain, Delta strain, Omicron strain, including but not limited to COVID-19 recombinant subunit vaccine and COVID-19 inactivated vaccine, a herpes zoster vaccine, an influenza vaccine, such as a quadrivalent influenza vaccine.
8. A pharmaceutical composition comprising an antigen and the composition of any one of items 1-5.
9. The pharmaceutical composition according to item 8, wherein the amount of the antigen is 5-200 µg/mL, preferably 5-80 µg/mL.
10. The pharmaceutical composition according to any one of items 8-9, wherein the amount of CpG ODN is 100-300 µg/mL.
11. The pharmaceutical composition according to any one of items 8-9, wherein the amount of aluminum in the aluminum adjuvant is 300-600 µg/mL.
12. The pharmaceutical composition of any one of items 8-11, which is a hepatitis B vaccine, a rabies vaccine, a COVID-19 recombinant subunit vaccine, a COVID-19 inactivated vaccine, a herpes zoster vaccine, an influenza vaccine, such as a quadrivalent influenza vaccine.
13. Use of the pharmaceutical composition of any one of items 8 to 12 in the preparation of a medicament for inducing an immune response against an antigen in a subject.
14. The use of item 13, wherein the pharmaceutical composition is for administration to the subject in an effective amount, preferably the pharmaceutical composition is for administration to the subject twice with an interval of 2-24 weeks.
15. A method for preparing the pharmaceutical composition according to any one of items 8-12, comprising mixing the composition of any one of items 1-5, an antigen and optionally a pharmaceutically acceptable carrier, resulting in a reaction of adsorbing, coupling and/or emulsification, and formulating an injection, an oral preparation, or a nasal spray therefrom.

The CpG ODN used in the present disclosure includes ODN 1-ODN 4, which respectively have the following nucleotide sequences:
ODN 1 (5'-tcgacgttcgtcgttcgtcgttc-3');
ODN 2 (5'-tcgcgacgttcgccgacgttcgta-3');
ODN 3 (5'-tcgtcgacgtcgttcgttctc-3');
ODN 4 (5'-tcgcgaacgttcgccgcgtacgtacgcgg-3');
wherein, the nucleotides of ODN 1-ODN 4 are all phosphorothioated nucleotides.

The present disclosure has the following beneficial technical effects:
(1) The CpG adjuvant provided by the present disclosure produces a synergistic effect when used in combination with an aluminum adjuvant, and the double-adjuvant vaccine shows better immunogenicity than a single adjuvant vaccine;
(2) The CpG adjuvant provided by the present disclosure can quickly induce the production of protective antibodies and reduce the number of immunizations when used in combination with an aluminum adjuvant. The method includes administering an effective amount of the pharmaceutical composition of the present disclosure to a subject. The method may further comprise administering the pharmaceutical composition to a subject in an effective amount, preferably the pharmaceutical composition is administered to the subject twice with an interval of 2-24 weeks. By administration of the pharmaceutical composition of the present disclosure to a subject twice, an immune response against the antigen in the subject can be induced and a protective antibody response level can be generated, which reduces the number of administrations and shortens the entire immunization period for inducing immune response in the subject as compared to the current method of inducing an immune response by administration for three times, and thus provides convenience and ensures subject compliance when rapid protection is required or subsequent doses cannot be guaranteed;
(3) The CpG adjuvant provided by the present disclosure can reduce the dosage of antigen, thereby reducing the safety hazards of viral antigens in production, and reducing the volume of subunit recombinant vaccine antigen sources, thereby expanding production capacity, reducing costs, and improving safety;
(4) The CpG adjuvant provided by the present disclosure can produce a strong and lasting protective effect when used in combination with an aluminum adjuvant.

### BRIEF DESCRIPTION OF THE DRAWINGS

Figure 1 shows the effects of different ODNs on hepatitis B vaccines.
Figure 2 shows the effect of ODN 1 on rabies vaccine.
Figure 3 shows the effect of ODN 1 on anti-SARS-CoV-2 RBD antibody titers in the serum of mice immunized with COVID-19 recombinant subunit vaccines.
Figure 4 shows the effect of ODN 1 on anti-SARS-CoV-2 S1 antibody titers in the serum of mice immunized with COVID-19 recombinant subunit vaccines.
Figure 5 shows the effect of ODN 1 on the titers of neutralizing antibodies against SARS-CoV-2 in the serum of mice immunized with COVID-19 recombinant subunit vaccines.
Figure 6 shows the effect of different concentrations of ODN 2 on anti-SARS-CoV-2 S 1 antibody titers in the serum of mice immunized with COVID-19 inactivated vaccines.
Figure 7 shows the effect of ODN 2 on anti-SARS-CoV-2 S1 antibody titers in the serum of mice immunized with COVID-19 inactivated vaccines containing different concentrations of antigens.
Figure 8 shows the specific titer of anti-SARS-CoV-2 S 1 protein in the serum of mice after immunization with COVID-19 recombinant subunit vaccines containing different ODNs.
Figure 9 shows the experimental results of Example 7, showing that there is a synergistic effect by the combination of ODN 1 and an aluminum hydroxide adjuvant, and the double-adjuvant group can significantly improve anti-gE protein titer as compared with the single aluminum adjuvant group and the single ODN 1 adjuvant group.

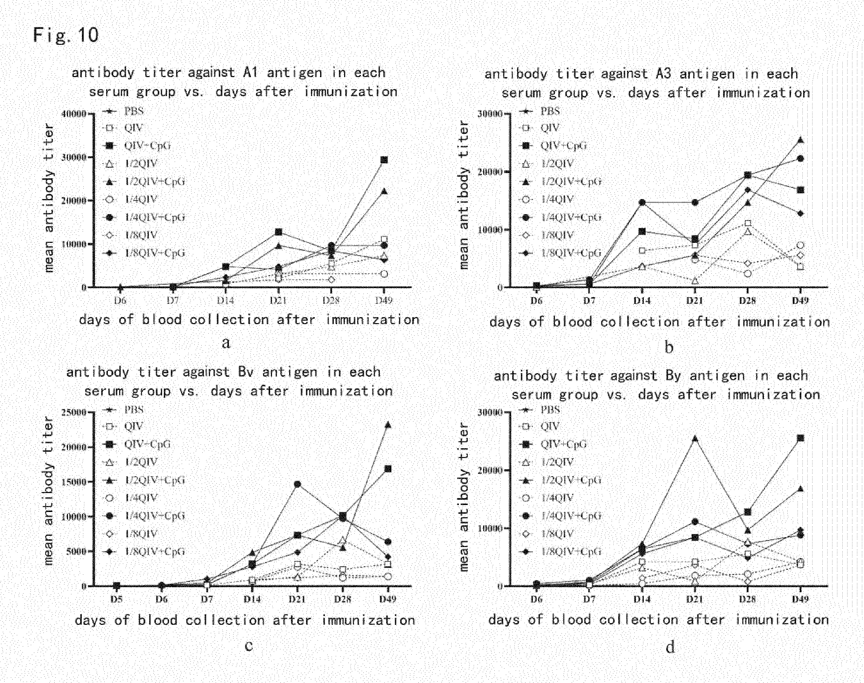

Figure 10 shows the experimental results of Example 8, including a graph (a) showing the relationship between antibody titers against A1 antigen in the serum of each group and the days after immunization, a graph (b) showing the relationship between antibody titers against A3 antigen in the serum of each group and the days after immunization, a graph (c) showing the relationship between antibody titers against Bv antigen in the serum of each group and the days after immunization, and a graph (d) showing the relationship between antibody titers against By antigen in the serum of each group and the days after immunization. As can be seen from Figure 10, compared with the vaccine group without ODN 1 adjuvant, the ODN 1 adjuvant vaccine group can quickly induce the production of protective antibodies, significantly improve geometric mean titers of antibodies against four antigens after immunization with quadrivalent split influenza vaccines.

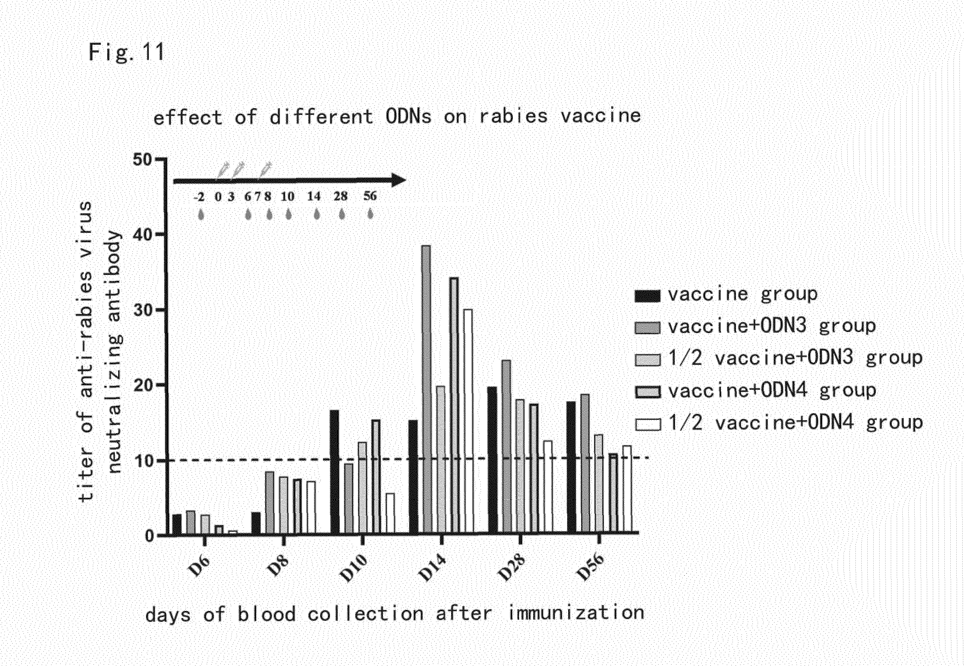

Figure 11 shows the experimental results of Example 9, wherein the experiment is divided into five groups: rabies vaccine, rabies vaccine + ODN 3, 1/2 rabies vaccine + ODN 3, rabies vaccine + ODN 4, and 1/2 rabies vaccine + ODN 4; the results shows that compared with the vaccine group without ODN 3 or ODN 4, the corresponding vaccine groups with ODN 3 or ODN 4 adjuvant can significantly increase neutralizing antibody titers of rabies vaccines. In addition, the vaccine groups with ODN 3 or ODN 4 adjuvant can achieve a higher immunization effect under 1/2 antigen content conditions than the groups with original antigen content.

### DETAILED DESCRIPTION OF THE INVENTION

In order to make the purpose, technical solution, and advantage of the present disclosure more clear, the present disclosure will be further described in detail below with reference to specific Examples and Figures.

Oligonucleotides are molecules formed by linkage of single nucleotides composed of a sugar (such as deoxyribose or ribose), a phosphate group, and a base; wherein the sugar molecule and the base are linked to form a nucleoside, which is then connected by a phosphate group to form a nucleotide. The bases forming nucleosides comprise pyrimidines and purines; wherein the pyrimidine includes a thymine (abbreviated as T or t) and a cytosine (abbreviated as C or c); the purine includes an adenine (abbreviated as A or a) and a guanine (guanine, abbreviated as G or g). Oligonucleotides can be single-stranded or double-stranded. In the present disclosure, "Oligodeoxynucleotide" (ODN) can be replaced by its English abbreviation ODN.

"Chemical modification": as compared with natural DNA, the oligonucleotides of the present disclosure can undergo various chemical modifications, and the sites of modification can be in the phosphodiester bonds between nucleosides, in ribose units or/and in bases (A, T, C, G, uracil, abbreviated as U or u). Modifications can be made during or after synthesis of the oligonucleotide. Chemical modifications during synthesis can be made internally or at the 5' end of the oligonucleotide. The synthesized oligonucleotide can be chemically modified, but is not limited to, at reactive groups (such as phosphate or hydroxyl groups at the 5' or 3' end). Specific methods of these chemical modifications can be understood by those skilled in the art. Chemical modifications in the present disclosure include backbone modifications of oligonucleotides, wherein at least one non-bridging phosphate oxygen atom in the internucleotide bond is replaced by a sulfur atom. The backbone of oligonucleotides can also be modified with non-ionic DNA analogues, such as alkyl and aromatic-carbon phosphate compounds (charged oxygen atoms of carbon phosphate compounds are replaced by alkyl and aromatic groups), or oxygen atoms in phosphodiesters and alkyl phosphate triesters are alkylated. The oligonucleotide may also be a chimera of phosphorothioyl and phosphodiester. Chemical modifications also include base substitutions, such as substitutions of C-5 propynepyrimidine or 7-deaza-7 substituted purine. Chemical modifications also include base modifications. Modified bases are chemically different from typical bases found in nature, but they have basic chemical structures of the naturally occurring bases. The oligonucleotides in the present disclosure can also be modified with cytosine derivatives or thymidine derivatives. Cytosine derivatives here refer to cytosine-like nucleosides (excluding cytosine). Thymidine derivatives refer to thymine-like nucleosides (excluding thymine). In addition, the modification of the oligonucleotide in the present disclosure also includes connection of a glycol (such as tetraethylene glycol or hexaethylene glycol) to both or one terminus of the oligonucleotide.

In one embodiment, the present disclosure provides a CpG ODN comprising or consisting of a nucleotide sequence selected from SEQ ID NOs: 1-4, wherein at least one nucleotide in the nucleotide sequence is a chemically modified nucleotide having a structure of formula (I): 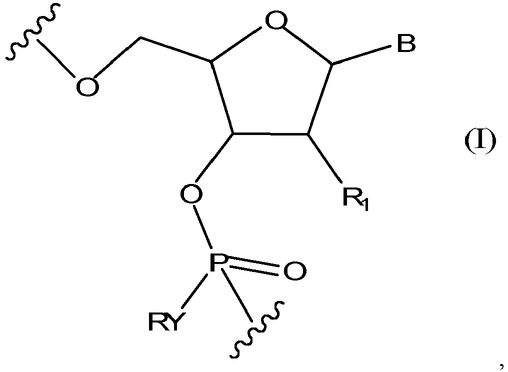 wherein, Y is S or O, R is H or a positively charged counterion, B is independently an unmodified or modified nucleobase, and R1 is H, F, Cl, OH, OMe, Me, O-ethyloxymethyl.

In another embodiment, Y is S. In another embodiment, all nucleotides in the nucleotide sequence of the CpG ODN are chemically modified nucleotides having the structure of formula (I). In another embodiment, the sequence of the CpG ODN is selected from SEQ ID NOs: 1-4, preferably, completely phosphorothioated SEQ ID NOs: 1-4.

In one embodiment, the present disclosure provides a composition comprising the CpG ODN described herein and a pharmaceutically acceptable carrier. The pharmaceutically acceptable carrier is a pharmaceutically acceptable carrier known in the art for use in compositions or vaccine compositions, such as phosphate buffer, etc. The pharmaceutically acceptable carrier may also comprise stabilizers for stabilizing the antigen, such as one or more of maltose, human serum albumin, vitamin C, cysteine, urea, gelatin, etc.

In one embodiment, the composition further comprises one or more other adjuvants that work together with the immunomodulatory CpG ODN, such as an aluminum adjuvant, such as an aluminum hydroxide and an insoluble aluminum salt colloid, an oil-water emulsion, a microorganism and a metabolite thereof, a nucleic acid and an analog thereof, a cytokine, an immunostimulatory complex, a propolis, and a liposome, preferably an aluminum adjuvant.

In one embodiment, the present disclosure discloses use of a CpG ODN of the present application in the preparation of an adjuvant. In one embodiment, the adjuvant is used for the preparation of a vaccine, preferably for the preparation of hepatitis B vaccine, rabies vaccine, COVID-19 recombinant subunit vaccine, and COVID-19 inactivated vaccine.

In one embodiment, the present disclosure provides a pharmaceutical composition comprising an antigen and a composition described herein.

In one embodiment of the pharmaceutical composition, the amount of the antigen is 5-200 µg/mL, preferably 5-80 µg/mL.

In one embodiment of the pharmaceutical composition, the amount of the CpG ODN is preferably 100-300 µg/mL.

In one embodiment of the pharmaceutical composition, the amount of aluminum adjuvant is preferably 300-600 µg/mL.

In one embodiment of the pharmaceutical composition, the pharmaceutical composition is hepatitis B vaccine, rabies vaccine, COVID-19 recombinant subunit vaccine, or COVID-19 inactivated vaccine.

In one embodiment, the present disclosure discloses use of the pharmaceutical composition of the present disclosure in the preparation of a medicament for inducing an immune response against an antigen in a subject. In a further embodiment, the use further includes re-administration of an effective dose of the pharmaceutical composition to the subject, with an interval of 2-24 weeks.

In one embodiment, the present disclosure discloses a method for preparing the pharmaceutical composition, comprising sequentially adding the CpG ODN and other adjuvants, antigens and pharmaceutically acceptable carriers at a dosage ratio into a container, resulting in a reaction of adsorbing, coupling and/or emulsification, and formulating an injection, an oral preparation, or a nasal spray therefrom.

In one embodiment of the method, when the other adjuvant is an aluminum adjuvant, such as aluminum hydroxide, the aluminum adjuvant is first mixed with the antigen to perform adsorption, and then mixed with the CpG ODN to perform adsorption.

CpG: C represents cytosine, G represents guanine, and p represents phosphodiester bond. CpG is an unmethylated dinucleotide composed of cytosine and guanine linked by a phosphodiester bond.

The term "adjuvant" refers to substances that are injected into animals prior to the antigen or mixed with the antigen at the same time, which can enhance the immunogenicity of the antigen. In animal vaccines, adjuvants can enhance the immune efficacy thereof.

### Aluminum adjuvant

The aluminum adjuvant described in the present disclosure is a well-known and commonly used immune adjuvant in the art, which can strongly adsorb protein antigens from the solution and form a precipitate. When inoculated into a subject, the aluminum adjuvant can form an "antigen library" and slowly release the antigen, thereby fully extending the action period of the antigen. The aluminum adjuvant can also promote a local (injection site) response of macrophages. The aluminum adjuvant of the present disclosure may be selected from the group consisting of aluminum hydroxide, aluminum phosphate and aluminum sulfate.

### Antigen

Antigens described in the present disclosure refers to substances that can induce an immune response in the body's immune system and can bind the products of the immune response (antibodies and/or effector cells) in vivo or in vitro to produce a specific reaction, which include proteins, lipids, carbohydrates, nucleic acids, compounds, etc.; wherein proteins as antigens include modified proteins (such as glycosylation or methylation; for example, the protein is cyclized or attached to lipids) or unmodified proteins. Antigens associated with infectious agents or diseases include antigens that are part of the infectious agent, such as envelope proteins, capsid proteins, surface proteins, toxins, cell walls, antigenic lipids, and the like. Other suitable antigens may include antigens of the host, including those induced, modified or overexpressed as markers of infection or disease. All such antigens derived from or associated with infectious agents, infections, conditions or diseases are suitable for use in the present disclosure.

In some embodiments of the present disclosure, antigens are antigens related to viruses including but not limited to hepatitis virus, rabies virus, SARS-CoV-2, etc.

The "vaccine" in the present disclosure is a vaccine well known to those skilled in the art, and generally refers to any biological products that can induce the production of specific antibody and/or cellular immunity against specific pathogens in an individual after inoculation by injection or mucosal route thereby conferring protection or ability of eliminating pathogens to the individual; the vaccines includes proteins, polysaccharides, nucleic acids, live vectors or infectious agents, etc. Vaccines are active immunization preparations for the prevention of infectious diseases, which are prepared from pathogenic microorganisms (such as bacteria, rickettsia, viruses, etc.) and metabolites thereof by artificially attenuating, inactivating or genetic engineering and other methods. Vaccines retain the properties of pathogenic bacteria in stimulating the immune system of animal body. When the animal is exposed to such innocuous pathogen, the immune system will produce some protective substances, such as immune hormones, active physiological substances, specific antibodies, etc.; when the animal is exposed to such pathogen again, the immune system of the animal will follow its original memory and produce more protective substances to prevent the damage of pathogenic bacteria.

### Examples

### Example 1 Effect of ODN 1-containing Hepatitis B Vaccine on the Immune Effect of Healthy Adults

### 1. Reagents

(1) Recombinant (Hansenula polymorpha) hepatitis B vaccine (CpG ODN adjuvant), produced by Yunnan Walvax Biotechnology Co., Ltd., vaccine specifications: 0.5 mL/vial, containing 10 µg of hepatitis B virus surface antigen, ODN 1 250 µg, aluminum content of 0.45-0.60 mg/mL (Batch number: 20151201);
(2) Recombinant (Hansenula polymorpha) hepatitis B vaccine, produced by Dalian Hissen Bio-pharm Co., Ltd., vaccine specifications: 0.5 mL/vial, containing 10 µg of hepatitis B virus surface antigen, and aluminum content of 0.35-0.62 mg/mL (Batch number: 201505082).

### 2. Methods

48 volunteers aged 18 to 60 who had been screened with negative hepatitis B virus (HBV) indicators, and normal liver and kidney functions and met the inclusion and exclusion criteria were selected as research subjects. A random, blind, and controlled design method was used to conduct the study. The subjects were divided into a test group and a control group with a ratio of 1:1, that is, 24 people in each group. The test group was vaccinated with the recombinant (Hansenula polymorpha) hepatitis B vaccine (CpG ODN adjuvant) produced by Yunnan Walvax Biotechnology Co., Ltd.; the control group was vaccinated with the recombinant (Hansenula polymorpha) hepatitis B vaccine produced by Dalian Hissen Bio-pharm Co., Ltd.. Each group was vaccinated according to the vaccination schedule at weeks 0, 4 and 24, with one dose of 0.5 mL per dose, administered by intramuscular injection at the skin of deltoid muscle on the lateral side of upper arm. The subjects after vaccination were observed and recorded at different times, and differences in the incidence of various adverse reactions between the two groups were compared. Blood samples were collected from all subjects before the first dose of vaccination and 30 days after the second and third doses of vaccination, and serum hepatitis B surface antibodies (anti-HBs) were detected by using chemiluminescence immunoassay. The blood samples were detected for HBsAg and anti-HBc again before the first dose of vaccination, and detected for anti-HBs concentration after each dose of vaccination. An antibody concentration of ≥10.0 mIU/mL was defined as antibody positive, and ≥1000.0 mIU/mL was defined as strong antibody positive. The differences in hepatitis B surface antibody (anti-HBs) positive rate and geometric mean concentration (GMC) between the two groups were compared.

### 3. Results

During the observation period, the incidence rate of adverse events was 66.67% (16 cases) in the test group, and 54.17% (13 cases) in the control group (*P*=0.556). All adverse events were grade 1 or 2, without adverse events of grade 3 or above. As shown in Table 1, the antibody GMC results after complete immunization showed that the antibody GMC in full analysis set (FAS set) in the test group was 2598.56 (95% *CI*: 1127.90-5986.90) mIU/mL, which was higher than 371.97 (164.54-840.91 ) mIU/mL in the control group; the antibody GMC in per protocol set (PPS set) in the test group was 7808.21 (3377.00-18052.00) mIU/mL, which was higher than [843.22 (95%CI: 213.80-3325.90) mIU/mL] in the control group. Anti-HBs positive rates in FAS (PPS) set were 95.83% (100.00%) in both test group and control group. The results of strong anti-HBs positive rate of the FAS (PPS) set showed 79.17% (90.00%) in the test group and 33.33% (50.00%) in the control group. The difference between groups was statistically significant (*P*=0.003) for FAS set, and the difference between groups was not statistically significant (*P*=0.074) for PPS set.

Conclusions: Compared with the control vaccine, CpG-containing hepatitis B vaccine had a better safety profile. Moreover, 30 days after the second and third doses of immunization, the antibody GMC of the test group was significantly higher than that of the control group, which showed that the double-adjuvanted hepatitis B vaccine (10 µg dose) containing both CpG adjuvant and aluminum adjuvant had better immunogenicity than the single aluminum-adjuvanted hepatitis B vaccine (10 µg dose) with the best immunization effect on the market. In addition, after the second dose of immunization, the test group could achieve a positive rate of 95%, that is, 2 doses of the test vaccine could achieve the effect of 3 doses of the control vaccine, indicating that inoculation of double-adjuvanted hepatitis B vaccines containing both CpG adjuvant and aluminum adjuvant could accelerate the production of hepatitis B antibodies after immunization, and rapid induction of protective antibodies had many advantages, especially in the case that rapid protection was required or subsequent doses could not be guaranteed. At the same time, in terms of vaccine cost and manpower, a two-dose vaccine schedule would cost less than a three-dose vaccine.

**Table 1 Comparison of antibody positive rate and GMC between the test group and the control group after vaccination with hepatitis B vaccine**

| indicators | | FAS | | | PPS | |
|---|---|---|---|---|---|---|
| | Test group (number) | control group (number) | *P* value | Test group (number) | control group (number) | *P* value |
| After the second dose of immunization | | | | | | |
| Antibody GMC^{a} | 350.27 (133.14-921.50) | 37.98 (14.65-98.48) | 0.002 | 265.21 (133.14-921.50) | 14.96 (14.65-98.48) | 0.008 |
| Positive rate^{b} | 95.83(23) | 79.17(19) | 0.188 | 100.00(10) | 66.67(8) | 0.096 |
| Strong Positive rate^{b} | 29.17(7) | 4.17(1) | 0.048 | 20.00(2) | 0.00(0) | 0.195 |

| After the third dose of immunization | | | | | | |
|---|---|---|---|---|---|---|
| Antibody GMC^{a} | 2598.56 (1127.90-5986.90) | 371.97 (164.54-840.91) | 0.001 | 7808.21 (3377.00-18052.00) | 843.22 (213.80-3325.90) | 0.008 |
| Positive rate^{b} | 95.83(23) | 95.83(23) | 1.000 | 100.00(10) | 100.00(12) | 1.000 |
| Strong Positive rate^{b} | 79.17(19) | 33.33(8) | 0.003 | 90.00(9) | 50.00(6) | 0.074 |

| | | | | | | |
|---|---|---|---|---|---|---|
| Note: ^{a}mIU/mL (95%*CI*), the difference between the two groups was compared by independent sample T test; ^{b}% (cases), the difference between the two groups was compared by Fisher's exact probability method; GMC: geometric mean concentration; FAS: Full Analysis Set; PPS: Per Protocle Set; strong positive: Hepatitis B surface antibody ≥1000 mIU/mL; test group: vaccinated with hepatitis B vaccine containing CpG adjuvant; control group: vaccinated with commercially available hepatitis B vaccine. | | | | | | |

### Example 2 Effects of different ODNs on hepatitis B vaccine

### 1. Materials

ODN 1, ODN 2 and positive control ODN 5 (5'-TGACTGTGAACGTTCGAGATGA-3', wherein all the nucleotides were completely phosphorothioated nucleotides) were provided by Jiangsu Taipuri Biotechnology Co., Ltd.;
Recombinant hepatitis B vaccine (CHO cells), specifications: 1.0 mL, 20 µg/tube, was purchased from North China Pharmaceutical Jintan Biotechnology Co., Ltd.;
BALB/c mice (SPF grade) were purchased from Zhejiang Charles river Experimental Animal Technology Co., Ltd.

### 2. Methods

Female BALB/c mice weighing 15-18 g with no history of drug administration were selected and divided into 5 groups. Each group was immunized once on D1 and D28 according to the designed immunization program by intramuscular administration to left tibial anterior, 0.1 mL each time. The mice were 5-6 weeks old when administered for the first time. According to the designed time, blood samples were collected into blood collection tubes through cardiac puncture or orbital blood collection, placed at room temperature for about 30-60 minutes and then centrifuged. All samples were centrifuged at 1800g for 15 minutes at 4°C to obtain serum. Anti-HBs antibody levels in serum samples were detected by a commercial hepatitis B surface antibody detection kit.

The mice were specifically grouped as follows:

| group | test substance | Administration volume (mL) | Hepatitis B vaccine concentration (µg/mL) | CpG adjuvant concentration (µg/mL) | Number of animals (F) | Time point of Blood collection |
|---|---|---|---|---|---|---|
| 1 | vehicle^{a} | 0.1 | 0 | 0 | 6 | 8W after administration |
| 2 | Hepatitis B Vaccine | 0.1 | 20 | 0 | 24 | 2W, 4W, 6W, 8W after administration |
| 3 | ODN 1+ Hepatitis B Vaccine | 0.1 | 10 | 125 | 24 | 2W, 4W, 6W, 8W after administration |
| 4 | ODN 2+ Hepatitis B Vaccine | 0.1 | 10 | 125 | 24 | 2W, 4W, 6W, 8 W after administration |
| 5 | ODN 5+ Hepatitis B Vaccine | 0.1 | 10 | 125 | 24 | 2W, 4W, 6W, 8W after administration |

| | | | | | | |
|---|---|---|---|---|---|---|
| Note: a, PBS; F, female; 6 mice per blood collection point. | | | | | | |

### 3. Results

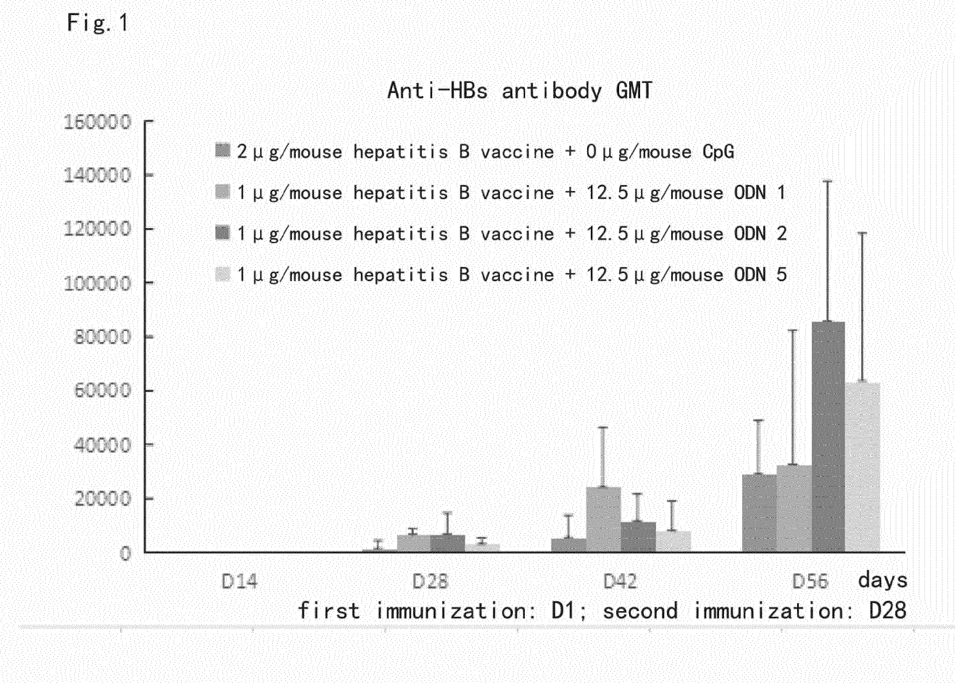

As shown in Figure 1, after the second immunization, the antibody titers produced in the 1/2 antigen groups (groups 3, 4, and 5) were significantly higher than that of the group without ODN (group 2). The antibody titer levels continued to increase 4 weeks, 6 weeks, and 8 weeks after the first immunization, and reached the highest antibody titer level in this experiment at week 8; wherein the antibody titers of mice in groups 4 and 5 increased strongly at week 8, indicating that antibody concentrations could maintain for a long time.

Conclusion: Different ODNs can reduce the dosage of hepatitis B vaccine antigen, and can achieve a strong and lasting protective effect when combined with hepatitis B vaccines.

### Example 3 Effect of ODN 1 on rabies vaccine

### 1. Materials

ODN 1, provided by Changchun Huapu Biotechnology Co., Ltd.;
Vero cell rabies vaccine, specification of 1 mL/tube (containing 2.5IU), obtained from Changchun Institute of Biological Products Co., Ltd.;
Aluminum hydroxide adjuvant, provided by Changchun Institute of Biological Products Co., Ltd.;
BALB/c mice were purchased from Charles river Experimental Animal Technology Co., Ltd.

### 2. Methods

Mice with a weight of 18-22 g and no history of drug administration were selected and divided into 8 groups, with 8 mice in each group, 4 male and 4 female. The specific groups were as follows: rabies vaccine, rabies vaccine + aluminum hydroxide, rabies vaccine + 1.25 µg ODN 1, rabies vaccine + 5 µg ODN 1, rabies vaccine + 20 µg ODN 1, rabies vaccine + aluminum hydroxide + 1.25 µg ODN 1, rabies vaccine + aluminum hydroxide + 5 µg ODN 1, rabies vaccine + aluminum hydroxide + 20 µg ODN 1, wherein the concentration of aluminum hydroxide was 0.45 mg/mL (counted as aluminum content).

The above rabies vaccine, ODN 1 and aluminum hydroxide adjuvant were diluted and dissolved in PBS at designed proportion. Immunization of mice: On day 0, day 7 and day 21, the mice were immunized respectively according to different groups. The immunization method was intraperitoneal injection, with 0.5 mL per injection. On day 4, day 10 and day 31, blood was collected from the tail vein of the mice, and the serum was separated. The rabies vaccine antibody titer in the serum of the mice was detected by a rapid rabies vaccine fluorescence foci inhibition test (RFFIT). Blood was collected from the tail vein of mice two days before immunization, and the obtained serum was used as a negative control.

### 3. Results

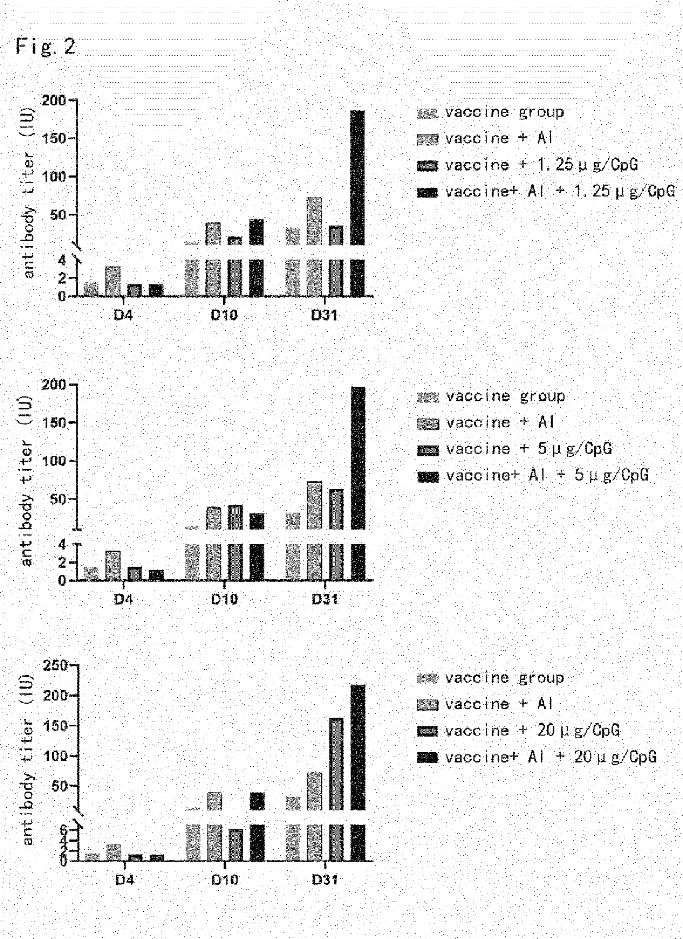

As shown in Figure 2, on day 31, the antibody titers of rabies vaccines in serum of mice were respectively 72.13, 35.79, 186.28, 63.1, and 197.63 IU for groups of rabies vaccine + aluminum hydroxide, rabies vaccine + 1.25 µg ODN 1, rabies vaccine + aluminum hydroxide + 1.25 µg ODN 1, rabies vaccine + 5 µg ODN 1 and rabies vaccine + aluminum hydroxide + 5 µg ODN 1, showing that there was a synergistic effect between ODN 1 and the aluminum hydroxide adjuvant; compared with the single aluminum hydroxide adjuvant group and the single ODN 1 adjuvant group, the double adjuvant group could significantly increase the antibody titers of rabies vaccines.

### Example 4 Effect of ODN 1 on COVID-19 recombinant subunit vaccine

### 1. Materials

(1) PBS phosphate buffer dry powder (Solarbio);
(2) SARS-CoV-2 S protein (Aero), specification of 500 µg/tube, batch number: C591P1-20B2F1-UF;
(3) SARS-CoV-2(COVID-19) S protein RBD (Aero), specification of 100 µg/tube, 100 mg/tube, batch number: C497P1-207AF1-TJ;
(4) ODN 1 (Jiangsu Taipuri Biotechnology Co., Ltd.), specification of 8.3 mg/mL, batch number: 202010001;
(5) BALB/c mice (SPF grade), provided by Zhejiang Charles river Experimental Animal Technology Co., Ltd.;
(6) Aluminum hydroxide, provided by Zhejiang Tianyuan biopharmaceutical Co., Ltd.

### 2. Methods

Female BALB/c mice weighing 15-18 g with no history of drug administration were selected and divided into 10 groups, wherein group 1 was a PBS vehicle control group, and groups 2-10 were test groups. Mice in each group were immunized by intraperitoneal injection on D0 and D28 according to designed immunization schedule, with 0.2 mL each time. The mice were 5-6 weeks old at the time of the first administration. According to the designed time, blood samples were collected via cardiac puncture or orbital blood collection on D7, D14, D21, D28, D56, and D84, placed at room temperature for about 30-60 minutes and then centrifuged. All samples were centrifuged at 1800g for 15 minutes at 4°C to obtain serum. Anti-S1 antibody and anti-RBD antibody levels in serum samples were detected by Sandwich ELISA.

The mice were specifically grouped as follows:

| Group | Antigen | | Adjuvant | | Number of immunized mice |
|---|---|---|---|---|---|
| | Types | Content µg/mL | Al content mg/mL | CpG content µg/mL | |
| Group 1 | - | 0 | 0 | 0 | 12 |
| Group2 | RBD | 25 | 0 | 0 | 30 |
| Group 3 | | 25 | 0.45 | 0 | 36 |
| Group 4 | | 7.5 | 0.45 | 100 | 36 |
| Group 5 | | 25 | 0.45 | 100 | 36 |
| Group 6 | | 75 | 0.45 | 100 | 30 |
| Group 7 | | 25 | 0 | 100 | 30 |
| Group 8 | S full-length antigen | 25 | 0.45 | 0 | 30 |
| Group 9 | | 25 | 0.45 | 100 | 30 |
| Group 10 | | 70 | 0.45 | 100 | 30 |

### 3. Results

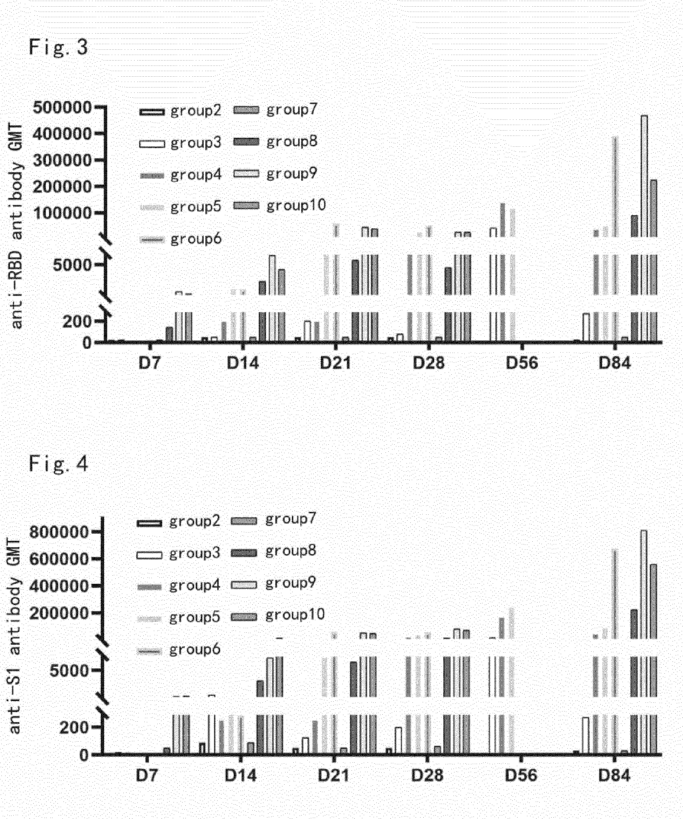

The results were shown in Figures 3 to 5. As can be seen from Figure 3, the anti-RBD antibody GMTs of mice in Group 3, Group 7 and Group 5 on day 14 were 50, 50 and 1270 respectively; the anti-RBD antibody GMTs on day 21 were 200, 50 and 10159 respectively; the anti-RBD antibody GMTs on day 28 were 79, 50 and 26390 respectively; the anti-RBD antibody GMTs on day 84 were 270, 52 and 51962 respectively. The above results showed that the anti-RBD antibody GMTs could be significantly increased in all double-adjuvant vaccine groups with RBD as the antigen as compared with that in corresponding adjuvant alone vaccine group, demonstrating that ODN 1 and aluminum hydroxide had a synergistic effect. The anti-RBD antibody GMTs on day 7 in mice in groups 8 and 9 were 141 and 800, respectively; the anti-RBD antibody GMTs on day 14 were 2425 and 6400 respectively; the anti-RBD antibody GMTs on day 21 were 5702 and 45948 respectively; the anti-RBD antibody GMTs on day 28 were 4525 and 25600 respectively; the anti-RBD antibody GMTs on day 84 were 90000 and 467654 respectively. The above results showed that the anti-RBD antibody GMTs could be significantly increased in double-adjuvant vaccine groups with S protein as the antigen as compared with that in corresponding Al-adjuvant alone vaccine group.

As can be seen from Figure 4, the anti-S 1 antibody GMTs on day 7 in mice in groups 8 and 9 were 50 and 400, respectively; the anti-S1 antibody GMTs on day 14 were 3200 and 7184 respectively; the anti-S1 antibody GMTs on day 21 were 6400 and 52780 respectively; the anti-S 1 antibody GMTs on day 28 were 12800 and 81275 respectively; the anti-S 1 antibody GMTs on day 84 were 224825 and 810000 respectively. The above results showed that the anti-S 1 antibody GMTs could be significantly increased in double-adjuvant vaccine groups with S protein as the antigen as compared with that in corresponding Al-adjuvant alone vaccine group.

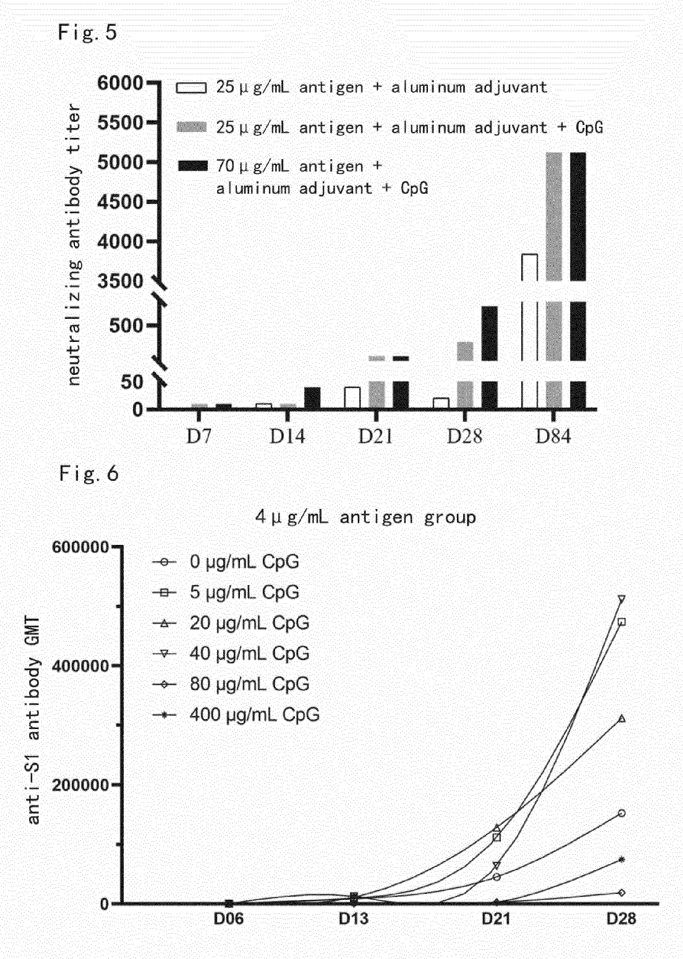

As can be seen from Figure 5, neutralizing antibody titers of mice in groups 8 and 9 on day 21 were 40 and 240, respectively; neutralizing antibody titers on day 28 were 20 and 360, respectively; neutralizing antibody titers on day 84 were 3840 and 5120 respectively. The above results showed that neutralizing antibody titers could be significantly increased in double-adjuvant vaccine groups with S protein as the antigen as compared with that in corresponding Al-adjuvant alone vaccine group.

When the aluminum content and ODN 1 content were maintained at 0.45 mg/mL and 100 µg/mL respectively, (1) as the increasing of antigen RBD content (groups 4-6), the anti-RBD antibody titer firstly increased and then decreased, showing that antibodies produced by vaccine groups with RBD as the antigen were increased in an antigen dose-dependent manner, and the levels of antibodies would be decreased when antigen concentrations exceeded a certain range. This experiment showed that the concentration of antibody production would decrease when the antigen concentration was above 70 µg/mL. The optimal antigen concentration was 25 µg/mL. (2) As the dose of antigen S full-length protein increased (groups 9-10), anti-S1 antibody titers increased, while neutralizing antibody titers remained stable, which showed that the levels of anti-S 1 antibody increased in an antigen dose-dependent manner, while the levels of neutralizing antibody did not change significantly as the antigen dose increased.

Conclusion: CpG adjuvant and aluminum adjuvant had a synergistic effect, which could significantly increase the anti-RBD antibody titer, anti-S 1 antibody titer and neutralizing antibody titer of the vaccine, and the protective effect was long-lasting. The combination of 5-100 µg/mL antigen with CpG adjuvant (100 µg/mL) and aluminum hydroxide (Al content of 0.45 mg/mL) produced better results. The increases of anti-S 1 antibody titer and anti-RBD antibody titer induced by CpG adjuvant were more significant in the group with S full-length protein as an antigen than that in the group with RBD as antigen.

### Example 5 Effect of ODN 2 on COVID-19 inactivated vaccine

### 1. Materials

(1) COVID-19 inactivated vaccine antigen was provided by Zhejiang Tianyuan biopharmaceutical Co., Ltd.;
(2) ODN 2, provided by Changchun Huapu Biotechnology Co., Ltd.;
(3) Aluminum hydroxide adjuvant, provided by Zhejiang Tianyuan biopharmaceutical Co., Ltd.;
(4) BALB/c mice were purchased from Charles river Experimental Animal Technology Co., Ltd.

### 2. Methods

18-20 g BALB/c mice were selected and grouped with 9 or 10 mice in each group (half male and half male); each group was immunized by intraperitoneal injection on D0 and D14 according to designed immunization program, with 0.5 mL per injection. According to the designed time, blood was collected at D0, D6, D13, D21 and D28 to separate serum, and all the sera were tested for S1 protein-specific IgGs and virus-neutralizing antibodies. Geometric mean titers of serum IgG and virus neutralizing antibodies in each group of animals were statistically calculated. The results were shown in Figures 6-7.

The specific groupings were as follows:

| Group | Antigen content (µg/mL) | Al content (mg/mL) | CpG content (µg/mL) | Number of immunized mice |
|---|---|---|---|---|
| G1 | 0 | 0.45 | 0 | 9 |
| G2 | | 0 | 400 | 9 |
| G3 | | 0.45 | 400 | 9 |
| G4 | 2 | 0.45 | 0 | 9 |
| G5 | | 0.45 | 5 | 9 |
| G6 | | 0.45 | 20 | 9 |
| G7 | | 0.45 | 40 | 9 |
| G8 | | 0.45 | 80 | 9 |
| G9 | | 0.45 | 400 | 9 |
| G10 | 4 | 0.45 | 0 | 9 |
| G11 | | 0.45 | 5 | 9 |
| G12 | | 0.45 | 20 | 9 |
| G13 | | 0.45 | 40 | 9 |
| G14 | | 0.45 | 80 | 9 |
| G15 | | 0.45 | 400 | 9 |
| G16 | 8 | 0.45 | 0 | 9 |
| G17 | | 0.45 | 5 | 9 |
| G18 | | 0.45 | 20 | 9 |
| G19 | | 0.45 | 40 | 9 |
| G20 | | 0.45 | 80 | 9 |
| G21 | | 0.45 | 400 | 9 |

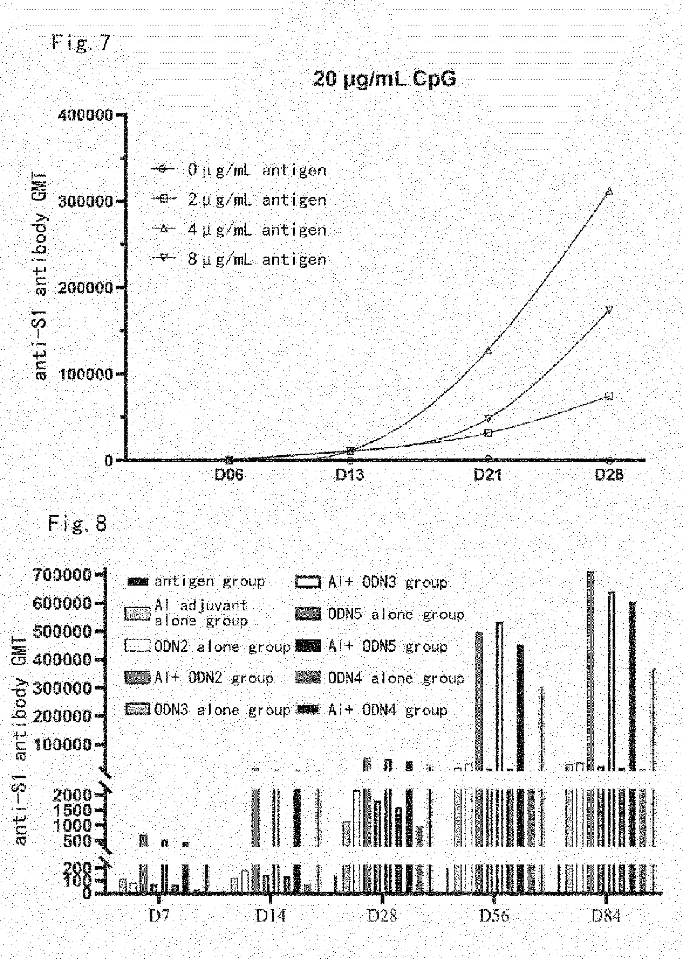

Results: As shown in Figure 6, when the antigen content of COVID-19 inactivated vaccine was 4 µg/mL, the combination of 5-80 µg/mL ODN 2 and aluminum hydroxide (Al content of 0.45 mg/mL) achieved a significant increase of anti-S 1 antibody GMT as compared with the groups without ODN 2. As shown in Figure 7, 20 µg/mL ODN 2 and aluminum hydroxide (Al content of 0.45 mg/mL) achieved a significant increase of anti-S 1 antibody GMT when used in COVID-19 inactivated vaccine containing 2-8 µg/mL of antigen.

Conclusion: The combination of ODN 2 and aluminum hydroxide could significantly increase the anti-S 1 antibody titer when used in COVID-19 inactivated vaccine. The optimal effect was achieved when the COVID-19 inactivated vaccine contained 5-80 µg/mL ODN 2, aluminum hydroxide (Al content of 0.45 mg/mL), and 2-8 µg/mL antigens.

### Example 6 Effect of different ODNs on COVID-19 recombinant subunit vaccine

### 1. Materials

(1) PBS phosphate buffer dry powder (Solarbio);
(2) SARS-CoV-2 S protein (Aero), specification of 500 µg/tube, batch number: C591P1-20B2F1-UF;
(3) ODN 2, ODN 3, ODN 4 and positive control ODN 5 (5'-TGACTGTGAACGTTCGAGATGA-3'), provided by Jiangsu Taipuri Biotechnology Co., Ltd.;
(4) Aluminum hydroxide adjuvant, purchased from Sinopharm;
(5) BALB/c mice were purchased from Charles river Experimental Animal Technology Co., Ltd.

### 2. Methods

Female BALB/c mice weighing 15-18 g with no history of drug administration were selected and divided into 11 groups, with approximately 30 mice in each group. Mice were immunized by intraperitoneal injection on D0 and D28, with 0.2 mL each time. The mice were 5-6 weeks old at the time of the first administration. According to the designed time, blood samples were collected via cardiac puncture or orbital blood collection on D7, D14, D28, D56, and D84 after administration, placed at room temperature for about 30-60 minutes and then centrifuged. All samples were centrifuged at 1800g for 15 minutes at 4°C to obtain serum. Anti-S1 antibody levels in serum samples were detected by Sandwich ELISA. The results were shown in Figure 8.

The specific groupings were as follows:

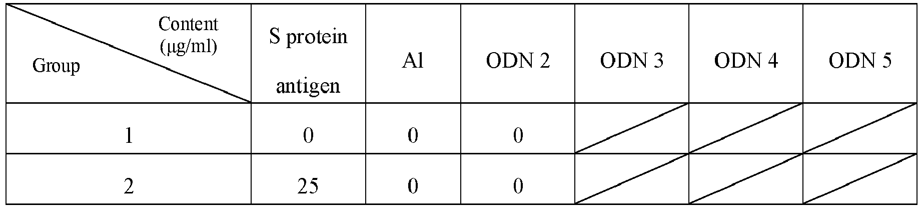

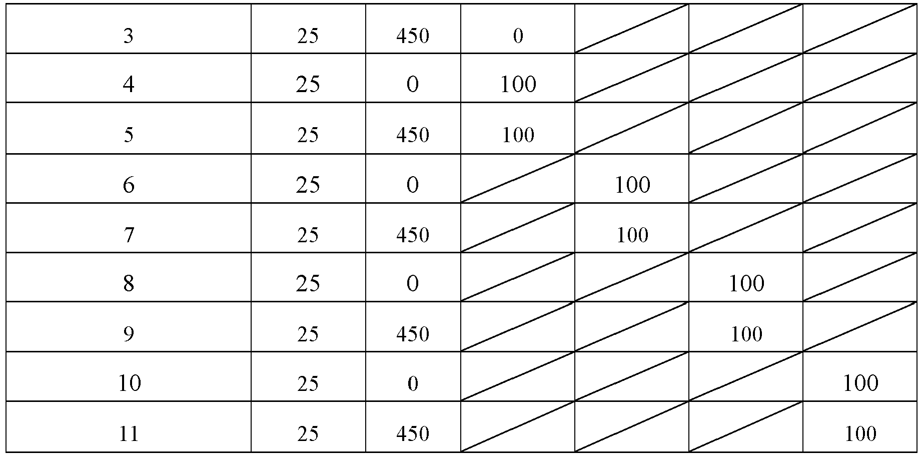

### 3. Results

As can be seen from Figure 8, anti-S 1 antibody GMTs on day 7 in mice in group 3 to group 11 were 111, 80, 700, 73, 550, 30, 317, 70 and 467 respectively; anti-S1 antibody GMTs on day 28 were 1120, 2133, 50667, 1813, 48316, 960, 29333, 1600 and 40333 respectively; anti-S1 antibody GMTs on day 84 were 28223, 34666, 709338, 22667, 641229, 10000, 373330, 16000 and 605329 respectively. The above results showed that there was a synergistic effect between different CpG ODNs and aluminum adjuvants; compared with single aluminum adjuvant group or single CpG ODN group, double adjuvant group could significantly increase the level of antibody titers, with long-lasting protective effect.

### Example 7 Effect of ODN 1 on Herpes Zoster Vaccine

### 1. Materials

PBS phosphate buffer dry powder, purchased from Solarbio;
ODN 1, provided by Jiangsu Taipuri Biotechnology Co., Ltd.;
Aluminum hydroxide adjuvant, purchased from Thermo;
Herpes zoster virus gE protein, purchased from SinoBiological;
BALB/c mice were purchased from Charles river Experimental Animal Technology Co., Ltd.

### 2. Methods

Mice with a weight of 18-22 g and no history of drug administration were selected and divided into 4 groups, with 8 mice in each group, 4 male and 4 female. The mice were specifically divided into the following groups: 10 µg gE protein group, 10 µg gE protein + 40 µg aluminum hydroxide, 10 µg gE protein + 5 µg ODN 1, 10 µg gE protein + 40 µg aluminum hydroxide + 5 µg ODN 1, wherein the content of aluminum hydroxide was counted as aluminum content.

gE antigen, aluminum hydroxide and ODN 1 were dissolved and diluted in PBS at designed ratios. According to the designed immunization program, mice in each group were immunized by intraperitoneal injection on D0, D28, and D56, with 0.1 mL per injection. According to designed schedule, blood was collected from the tail vein of mice on D0, D7, D14, D21, D28, D35, D42, D49, D56, D63, D70 and D77 to separate the serum, and anti-gE protein titers in the mouse serum were detected by ELISA. The serum obtained 7 days before immunization was used as a negative control.

### 3. Results

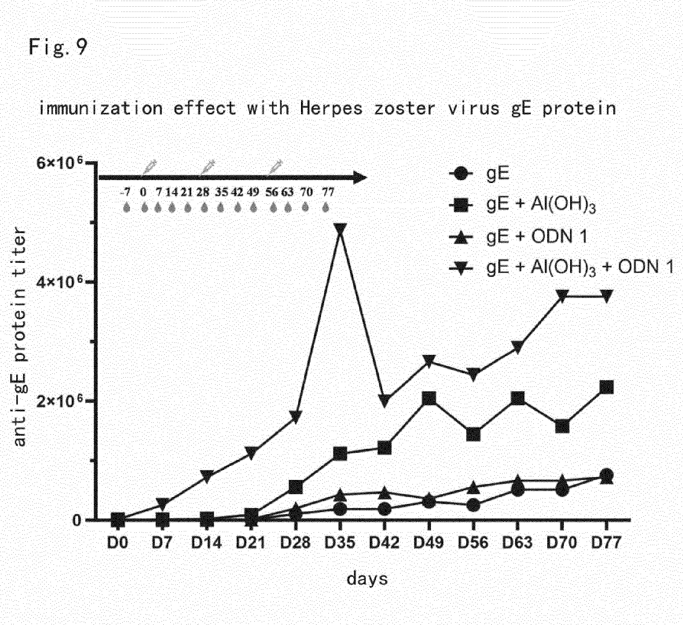

As shown in Figure 9, on day 35, anti-gE protein titers in serum of mice were respectively 190207, 1116680, 430539, and 4870992 for groups of 10 µg gE protein, 10 µg gE protein + 40 µg aluminum hydroxide, 10 µg gE protein + 5 µg ODN 1, 10 µg gE protein + 40 µg aluminum hydroxide + 5 µg ODN 1, showing that there was a synergistic effect between ODN 1 and the aluminum hydroxide adjuvant; compared with the single aluminum hydroxide adjuvant group and the single ODN 1 adjuvant group, the double adjuvant group could significantly increase the anti-gE protein titer.

### Example 8 Effect of ODN 1 on quadrivalent influenza vaccine

### 1. Materials

PBS phosphate buffer dry powder, purchased from Solarbio;
ODN 1, provided by Jiangsu Taipuri Biotechnology Co., Ltd.;
A1 influenza antigen, purchased from SinoBiological;
A3 influenza antigen, purchased from SinoBiological;
By influenza antigen, purchased from SinoBiological;
Bv influenza antigen, purchased from SinoBiological;
BALB/c mice were purchased from Charles river Experimental Animal Technology Co., Ltd.

### 2. Methods

Female mice weighing 15-20 g and with no history of drug administration were selected and divided into 9 groups. Groups 1 to 9 were respectively PBS group, quadrivalent split vaccine group (QIV), quadrivalent split vaccine + CpG group (QIV + CpG), 1/2 quadrivalent split vaccine group (1/2 QIV), 1/2 quadrivalent split vaccine + CpG group (1/2 QIV + CpG), 1/4 quadrivalent split vaccine group (1/4 QIV), 1/4 quadrivalent split vaccine + CpG group (1/4 QIV + CpG), 1/8 quadrivalent split vaccine group (1/8 QIV), 1/8 quadrivalent split vaccine + CpG group (1/8 QIV + CpG), 20 mice per group (10 mice in PBS group).

The above four influenza antigens (A1, A3, By, Bv) and ODN 1 were added into PBS at designed proportions and then dissolved for immunization of mice. On D0, the mice in each group were immunized once via intramuscular injection on the inner thigh of the hind limbs, with an injection volume of 0.1 mL. 0.1 mL solution used to immunize each mouse in the QIV group contained 3.5 µg of each of the four antigens; 0.1 mL solution used to immunize each mouse in the QIV + CpG group contained 3.5 µg of each of the four antigens and 5 µg of ODN 1; 0.1 mL solution used to immunize each mouse in the 1/2 QIV group contained 1.75 µg of each of the four antigens; 0.1 mL solution used to immunize each mouse in the 1/2 QIV + CpG group contained 1.75 µg of each of the 4 antigens and 5 µg of ODN 1; 0.1 mL solution used to immunize each mouse in 1/4 QIV group contained 0.875 µg of each of the four antigens; 0.1 mL solution used to immunize each mouse in the 1/4 QIV + CpG group contained 0.875 µg of each of the four antigens and 5 µg of ODN 1; 0.1 mL solution used to immunize each mouse in 1/8 QIV group contained 0.44 µg of each of the four antigens; 0.1 mL solution used to immunize each mouse in the 1/8 QIV + CpG group contained 0.44 µg of each of the four antigens and 5 µg of ODN 1; Animals were observed until D49 after immunization.

According to the experimental designed time, blood was collected from the mice in each test group (groups 2 to 9) on D4, D5, D6, D7, D14, D21, D28, and D49, and blood was collected from the mice in control group (group 1) on D7 and D28. Blood was collected in five mice in each group for each blood collection time point. Blood was collected from orbital venous plexus on D4, D5, D6, and D7 in groups 2 to 9, and blood was collected from eyeballs on D14, D21, D28, and D49. For all mice in group 1, blood was collected from eyeballs, and animals after eyeball removal and blood collection died via planned bleeding. The whole blood was placed at room temperature for about 30-60 minutes and then centrifuged. All samples were centrifuged at 1800g for 15 minutes at 4°C to obtain serum. The geometric mean titers of four antibodies in the mouse serum were detected by ELISA. The day before immunization, the serum in 10 mice before immunization was collected by enucleating eye balls as a negative control.

### 3. Results

In Figure 10, as can be seen from the geometric mean titers of the four antibodies in the serum of mice from D4 to D49 after immunization, the ODN 1 adjuvant vaccine group could quickly induce the production of protective antibodies as compared with the vaccine group without ODN 1 adjuvant. The vaccine groups added with CpG could significantly increase antibody geometric mean titers of the four antigens after immunization with quadrivalent split influenza vaccines, approximately 1-9 times higher than that in the group without CpG. In addition, under 1/8 antigen content conditions, the vaccine groups with CpG could achieve an immunization effect no less than that of in groups with original antigen content.

In summary, ODN 1 adjuvant could quickly induce the production of protective antibodies; significantly increase the antibody titer of quadrivalent influenza vaccine; reduce the amount of antigens and improve the safety of the vaccine.

### Example 9 Effect of different ODNs on rabies vaccine

### 1. Materials

ODN 3 and ODN 4 were provided by Jiangsu Taipuri Biotechnology Co., Ltd.;
Vero cell rabies vaccine, specification of 1 mL/tube (containing 2.5IU), obtained from Changchun Institute of Biological Products Co., Ltd.;
BALB/c mice were purchased from Charles river Experimental Animal Technology Co., Ltd.

### 2. Methods

Mice with a weight of 18-22 g and no history of drug administration were selected and divided into 5 groups, with 8 mice in each group, 4 male and 4 female. Groups 1-5 were as follows: rabies vaccine, rabies vaccine + ODN 3, 1/2 rabies vaccine + ODN 3, rabies vaccine + ODN 4, 1/2 rabies vaccine + ODN 4.

The above Vero cell rabies vaccine, ODN 3 and ODN 4 were diluted and dissolved in PBS according to designed proportion, and the Vero cell rabies vaccines in groups 1, 2 and 4 were diluted 100 times; the Vero cell rabies vaccines in groups 3 and 5 were diluted 200 times for immunization of mice. According to designed procedures, mice in each group were immunized once on day 0, day 3 and day 7, respectively. The immunization method is intramuscular injection into the hind legs of mice, with an injection volume of 0.1 mL. The contents of ODN 3 or ODN 4 in the 0.1 mL solution for immunization of mice in groups 2-5 were 5 µg. On day 4, day 6, day 8, day 10, day 14, day 28, and day 56, blood was collected from the tail vein of the mice, and the serum was separated. Titers of anti-rabies virus neutralizing antibody in the serum of the mice were detected by the rapid rabies vaccine fluorescence foci inhibition test (RFFIT). Blood was collected from the tail vein of mice two days before immunization, and the obtained serum was used as a negative control.

### 3. Results

As shown in Figure 11, on day 14, the titers of anti-rabies virus neutralizing antibody in the serum of mice in groups of rabies vaccine, rabies vaccine + ODN 3, 1/2 rabies vaccine + ODN 3, rabies vaccine + ODN 4, and 1/2 rabies vaccine + ODN 4 were 15.19, 38.33, 19.63, 34.07, and 29.83 respectively; the results shows that compared with the vaccine group without ODN 3 or ODN 4, the corresponding vaccine groups with ODN 3 or ODN 4 adjuvant could significantly increase neutralizing antibody titers of rabies vaccines. In addition, the vaccine groups with ODN 3 or ODN 4 adjuvant could achieve a higher immunization effect under 1/2 antigen content conditions than the groups with original antigen content.

### Equivalent technical solutions

The specific Examples described above further describe the purpose, technical solutions and beneficial effects of the present disclosure in detail, but they should not be construed as limiting the scope of the invention. It should be noted that any modifications, equivalent replacements and improvements made by those skilled in the art within the spirit and scope of the invention should be included within the protection scope of the present disclosure.

## Claims

1. A composition comprising CpG ODN and one or more other adjuvants that work together with the immunomodulatory CpG ODN, such as an aluminum adjuvant, such as an aluminum hydroxide and an insoluble aluminum salt colloid, an oil-water emulsion, a microorganism and a metabolite thereof, a nucleic acid and an analog thereof, a cytokine, an immunostimulatory complex, a propolis, and a liposome, preferably an aluminum adjuvant, wherein the CpG ODN comprises or consists of a nucleotide sequence selected from SEQ ID NOs: 1-4, wherein at least one nucleotide in the nucleotide sequence is a chemically modified nucleotide having a structure of formula (I): 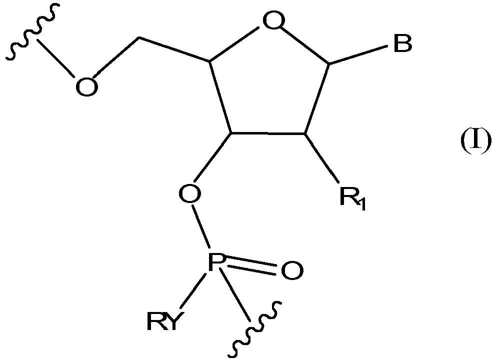 wherein, Y is S or O, R is H or a positively charged counterion, B is independently an unmodified or modified nucleobase, and Ri is H, F, Cl, OH, OMe, Me, O-ethyloxymethyl.

2. The composition of claim 1, wherein Y is S.

3. The composition of claim 1 or 2, wherein all nucleotides in the nucleotide sequence of the CpG ODN are chemically modified nucleotides having the structure of formula (I).

4. The composition of claim 3, wherein the sequence of the CpG ODN is selected from SEQ ID NOs: 1-4, preferably, completely phosphorothioated SEQ ID NOs: 1-4.

5. The composition of any one of claims 1-4, wherein the amount of CpG ODN is 10-500 µg/mL, and the amount of aluminum in the aluminum adjuvant is 300-600 µg/mL.

6. Use of the composition of any one of claims 1-5 in the preparation of a vaccine.

7. The use of claim 6, wherein the vaccine is a hepatitis B vaccine, a rabies vaccine, a COVID-19 vaccine, such as a COVID-19 vaccine against SARS-CoV-2 Alpha strain, Beta strain, Gamma strain, Delta strain, Omicron strain, including but not limited to a COVID-19 recombinant subunit vaccine and a COVID-19 inactivated vaccine, a herpes zoster vaccine, an influenza vaccine, such as a quadrivalent influenza vaccine.

8. A pharmaceutical composition comprising an antigen and the composition of any one of claims 1-5.

9. The pharmaceutical composition according to claim 8, wherein the amount of the antigen is 5-200 µg/mL, preferably 5-80 µg/mL.

10. The pharmaceutical composition according to any one of claims 8-9, wherein the amount of CpG ODN is 100-300 µg/mL.

11. The pharmaceutical composition of any one of claims 8-9, wherein the amount of aluminum in the aluminum adjuvant is 300-600 µg/mL.

12. The pharmaceutical composition of any one of claims 8-11, which is a hepatitis B vaccine, a rabies vaccine, a COVID-19 recombinant subunit vaccine, a COVID-19 inactivated vaccine, a herpes zoster vaccine, an influenza vaccine, such as a quadrivalent influenza vaccine.

13. Use of the pharmaceutical composition of any one of claims 8-12 in the preparation of a medicament for inducing an immune response against the antigen in a subject.

14. The use of claim 13, wherein the pharmaceutical composition is for administration to the subject in an effective amount, preferably the pharmaceutical composition is for administration to the subject twice with an interval of 2-24 weeks.

15. A method for preparing the pharmaceutical composition of any one of claims 8-12, comprising mixing the composition of any one of claims 1-5, the antigen and optionally a pharmaceutically acceptable carrier, resulting in a reaction of adsorbing, coupling and/or emulsification, and formulating an injection, an oral preparation, or a nasal spray therefrom.
